# EUROPEAN PATENT APPLICATION

(11) **EP 1 258 493 A1**
(43) Date of publication of application: **20.11.2002**
(21) Application number: 01111774.4
(22) Date of filing: 15.05.2001
(51) Int. Cl.: C07K 14/39, C07K 14/47, C12N 15/11, C12N 15/62

(54) **Cleavage and polyadenylation complex of precursor mRNA**

(71) Applicant: CELLZOME AG, 69117 Heidelberg (DE)
(72) Inventor: Bauer, Andreas, Dr., 69123 Heidelberg (DE); Gavin, Anne-Claude, Dr., 69181 Leimen (DE); Marzioch, Martina, 69126 Heidelberg (DE); Schultz, Jörg, Dr., 69126 Heidelberg (DE); Brajenovic, Miro, 69117 Heidelberg (DE); Grandi, Dr. Paolo, 69117 Heidelberg (DE)
(74) Representative: Grünecker, Kinkeldey, Stockmair & Schwanhäusser Anwaltssozietät

(57) **Abstract**

The present invention relates to novel components of the cleavage/polyadenylation machinery of precursor mRNA as well as to the complex containing the new components and its use. The complex is obtained by using one component thereof as a bait and isolating a highly organised complex consisting of at least 13 distinct proteins.

## Description

The present invention relates to components of the cleavage/polyadenylation machinery of pre-cursor mRNA, the complete protein complex, uses of said components and complex as well as to methods of preparing same.

Polyadenylation of precursor mRNA (pre-mRNAs) is an obligatory step in the maturation of most eukaryotic transcripts. The addition of poly(A) (polyadenosine) tails promote transcription termination and export of the mRNA from the nucleus. Furthermore, the poly(A) tails have the function to increase the efficiency of translation initiation and to help to stabilize mRNAs. Polyadenylation occurs posttranscriptionally in the nucleus of eukaryotic cells in two tightly coupled steps: the endonucleolytic cleavage of the precursor and the addition of a poly(A) tail.

In the yeast Saccharomyces cerevisiae, the pre-mRNA 3'-end processing signals are not as well conserved as in mammalian cells (see below). In addition to the cleavage and polyadenylation site, two cis-acting elements, called the efficiency element and the positioning element, are found upstream of the cleavage site. Efficiency elements contain the sequence UAUAUA (or close variants thereof) and are often repeated. The sequence AAUAAA and several related sequences can function as a positioning element.

Fractionation of yeast extracts led to the separation of protein factors that are required for mRNA 3'-end formation in vitro. The cleavage reaction requires cleavage factors I and II (CF I and CF II), whereas polyadenylation involves CF I, polyadenylation factor I (PF I) and poly(A) polymerase (Pap1).

CF I can be separated into two activities, CF IA and CF IB. CF IA is needed for both processing steps and is a heterotetrameric protein with subunits of 38, 50, 70 and 76 kDa that are encoded by the RNA5, CLP1, PCF11 and RNA14 genes. Rna14 shares significant sequence similarity to the 77 kDa subunit of mammalian cleavage stimulation factor (CstF) and Rna15 contains a RNA-binding domain homologous to that of the 64 kDa subunit of CstF.

In addition to the above mentioned four CFI subunits, Pab1 (poly(A) binding protein) was identified in purified CFI fractions. Both biochemical and genetic data indicate an involvement of Pab1 in poly(A) length control. CF IB consists of a single protein called Nab4/Hrp1 and is required for cleavage site selection and polyadenylation. A multiprotein complex which has CFII-PF I (= CPF) activity consists of nine polypeptides: Pap1 (poly(A) polymerase), Pta1, Pfs1, Pfs2, Fip1, Cft1/Yhh1, Cft2/Ydh1, Ysh1/Brr5, and Yth1. Pap1, a 64 kDa protein, was the first component of the yeast 3'-end formation complex to be purified to homogeneity. Pta1 is a 90 kDa protein which is required for both cleavage and polyadenylation of mRNA precursors. Pfs2 is a 53 kDa protein that contains seven WD40 repeats. Pfs2 has been shown to directly interact with subunits of CFII-PF1 and CFIA and is thought to function in the assembly and stabilization of the 3'-end processing complex. Fip1 has been demonstrated to physically interact with Pap1, Yth1 and Rna14 and it is believed that it tethers Pap1 to its substrate during polyadenylation. Cft1/Yhh1, Cft2/Ydh1, Ysh1/Brr5, and Yth1 are the counterparts of the four subunits of the mammalian cleavage and polyadenylation specificity factor, CPSF160, CPSF100, CPSF73 and CPSF30, respectively.

For the mammalian system, various data have been presented which have given evidence both for a conserved mechanism and also showed some differences between the yeast and the mammalian structures.

The composition and function of the mammalian complex based on the data to date is as follows:

The cleavage and polyadenylation factor (CPSF) is composed of 4 subunits: CPSF160 (involved in mRNA and poly(A) polymerase (PAP) binding), CPSF100, CPSF 73 and CPSF30 (involved in mRNA and PABII binding).

CPSF binds the AAUAAA hexanucleotides. CPSF links the mRNA 3'-end processing to the transcription. CPSF exists as a stable complex with the transcription factor TFIID complex. The 160 kDa subunit of CPSF binds to several hTAFII. TFIID recruits CPSF to the RNA polymerase II pre-initiation complex. Upon transcriptional activation CPSF dissociates from TFII and associates with the elongating RNA pol II (CTD carboxy-terminal domain of the largest subunit of the RNA polymerase II). CPSF is thought to travel with RNA pol II until they reach the polyadenylation site, where CPSF can bind the AAUAAA element. CPSF is required for the termination of transcription.

The interaction between CPSF and the AAUAAA element is weak and not so specific. The binding of CPSF to the hexanucleotide is greatly enhanced by a 2nd component of the polyadenylation machinery, the cleavage stimulation factor (CstF), which binds the G-U rich motif. CstF also binds the RNA pol II through its 50 kDa subunit (CstF50). Furthermore, CstF50 binds another component of the transcriptional machinery: BRCA1 associated RING domain protein (BARD1). BARD1 also interacts with RNA pol II. BARD1-CstF50 interaction inhibits polyadenylation in vitro and may prevent inappropriate mRNA processing during transcription. CstF is composed of 3 subunits: CstF64 (binds mRNA and symplekin (yeast homolog: Pta1), CstF77 (binds CPSF160, CstF64, CstF50) and CstF50 (binds RNA pol II and BARD1). The co-operative binding of CPSF and CstF to the polyadenylation site forms a ternary complex, which functions to recruit the other components of the polyadenylation machinery to the cleavage site: the two cleavage factors (CFIm and CFIIm) and the poly(A) polymerase (PAP).

CFIm is an heterodimer of 4 subunits 72, 68, 59, 25 components: one essential, CFIImA and one stimulatory, CFIIB. CFIImA contains hPCF11p and hClp1p (binds cPSF and CF I). CF IImB contains no factors previously shown to be involved in 3'-end processing and may be a new 3'-end processing factor. Although the identity of the proteins that perform the cleavage step is still unknown, it is well established that both CFIm and CFIIm are required. The reaction products of the cleavage suggest that a metal ion is involved. Surprisingly, PAP (but not its catalytic activity) is required for the cleavage.

After the cleavage step CstF, CFIm and CFIIm are dispensable. PAP bound to CPSF (through its 160 kD subunit) can start polyadenylating the cleaved 3'-end, but at that step, the process is very inefficient. The poly(A) binding protein II (PAB II) can bind the nascent poly(A) chain as soon as it reaches a minimal length of 10 poly(A). PAB II also interacts with the CPSF30. The binding of PAB II greatly stabilizes PAP at the 3'-end of the mRNA, supporting the progressive synthesis of a long poly(A) tail. In the nucleus, the length of the poly(A) tail is restricted to about 250 poly(A). This size restriction is probably achieved through stoichiometric binding of multiple PAB II. It is not yet known how the incorporation of a certain amount of PAB II in the complex terminates processive elongation.

CstF is part of the mammalian 3'-end processing complex and is a heterotrimeric protein with subunits of 77, 64 and 50 kDa. CstF-50 has been shown to interact with the BRCA1-associated protein BARD1 and this interaction suppresses the nuclear mRNA polyadenylation machinery in vivo. In a recent study it was found that treatment of cells with DNA damage-inducing agents causes a transient, but specific, inhibition of mRNA 3'-end processing in cell extracts. This inhibition reflects the BARD1/CstF interaction and involves enhanced formation of a CstF/BARD1/BRCA1 complex. A tumor-associated germline mutation in BARD1 decreases binding to CstF-50 and renders the protein inactive in polyadenylation inhibition. These results support the existence of a link between mRNA 3'-end formation and DNA repair/tumor suppression. The in vivo function of these interactions may be to inhibit the cleavage and polyadenylation of pre-mRNAs on polymerase molecules that are stalled at sites of DNA repair.

Cleavage stimulation factor (CstF) is one of the multiple factors required for mRNA polyadenylation in mammalians. CstF-64 may play a role in regulating gene expression and cell growth in B cells. The concentration of one CstF subunit (CstF-64) increases during activation of B cells, and this is sufficient to switch IgM heavy chain mRNA expression from membrane- bound to secreted form. Reduction in CstF-64 causes reversible cell cycle arrest in G0/G1 phase, while depletion results in apoptotic cell death.

In contrast to what is observed in yeast, the sequence elements in mammals, which specify the site of cleavage and polyadenylation, flank the site of endonucleolytic attack. One is the hexanucleotide AAUAAA found 10-30 bases upstream of the cleavage/polyadenylation site. The second is a G-U-rich motif located 20-40 bases downstream of the
cleavage/polyadenylation site. These two elements and their spacing determine the site of cleavage/polyadenylation and also the strength of the polyadenylation signal.

Some other elements, like sequences upstream of the AAUAAA (upstream sequence elements, USEs) play regulatory roles.

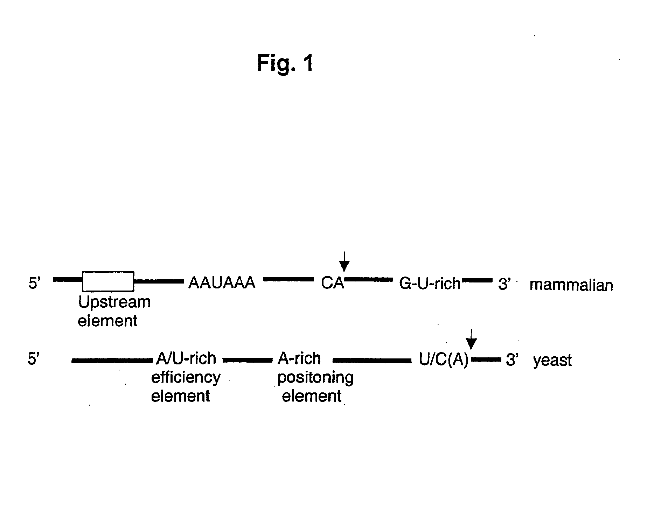

A schematic presentation of the motifs underlying mammalian polyadenylation and yeast polyadenylation are shown in Fig. 1. A review on the formation of mRNA 3'-ends in eukaryotes is given in Zhao, Hyman and Moore in Microbiology and Molecular Biology Reviews, 1999, pp. 405-445. A comparison of mammalian and yeast pre-mRNA 3'-end processing is also given in Keller and Minvielle-Sebastia in Nucleus and gene expression in Current Opinion in Cell Biology, 1997, Vol. 9, pp. 329-336.

There are diseases which involve defects in the function of the polyadenylation machinery.

Many viruses interact directly with components of the mRNA processing machinery.
The herpes simplex virus type 1 (HSV-1) immediate early (alpha) protein ICP27 is an essential regulatory protein that is involved in the shutoff of host protein synthesis,. It affects mRNA processing at the level of both polyadenylation and splicing. During polyadenylation, ICP27 appears to stimulate 3' mRNA processing at selected poly(A) sites. The opposite effect occurs on host cell splicing. That is, during HSV-1 infection, an inhibition in host cell splicing requires ICP27 expression. This contributes to the shutoff of host protein synthesis by decreasing levels of spliced cellular mRNAs available for translation. A redistribution of splicing factors regulated by ICP27 has also been seen.

Epstein-Barr virus BMLF1 gene product EB2 seems to affect mRNA nuclear export of intronless mRNAs and pre-mRNA 3' processing. EB2 contains an Arg-X-Pro tripeptide repeated eight times, similar to that described as an mRNA-binding domain in the herpes simplex virus type 1 protein US11.

Interestingly, both viruses have been found to precede the onset of lymphomas.

Influenza A virus NS1A protein binds the 30 kDa subunit of the cleavage and polyadenylation specificity factor (CPSF), NS1 protein (NS1A protein) via its effector domain targets the poly(A)-binding protein II (PABII) of the cellular 3'-end processing machinery. In vitro the NS1A protein binds the PABII protein, and in vivo causes PABII protein molecules to relocalize from nuclear speckles to a uniform distribution throughout the nucleoplasm. In vitro the NS1A protein inhibits the ability of PABII to stimulate the processive synthesis of long poly(A) tails catalyzed by poly(A) polymerase (PAP). Such inhibition also occurs in vivo in influenza virus-infected cells. Consequently, although the NS1A protein also binds the 30 kDa subunit of the cleavage and polyadenylation specificity factor (CPSF), 3' cleavage of some cellular pre-mRNAs still occurs in virus-infected cells, followed by the PAP-catalyzed addition of short poly(A) tails. Subsequent elongation of these short poly(A) tails is blocked because the NS1A protein inhibits PABII function. The NS1 effector domain functionally interacts with the cellular 30 kDa subunit of CPSF, an essential component of the 3' end processing machinery of cellular pre-mRNAs.

Metachromatic leukodystrophy (MLD) is a lysosomal storage disorder caused by the deficiency of arylsulfatase A (ASA). A substantial ASA deficiency has also been described in clinically healthy persons, a condition for which the term pseudodeficiency was introduced. The mutations characteristic for the pseudodeficiency (PD) allele have been identified. Sequence analysis revealed two A-G transitions. One of them changes the first polyadenylation signal downstream of the stop codon from AATAAC to AGTAAC. This causes a severe deficiency of a 2.1-kilobase (kb) mRNA species. The deficiency of the 2.1-kb RNA species provides an explanation for the diminished synthesis of ASA seen in pseudodeficiency fibroblasts.

MLD patients have been identified who are homozygous for the ASA-PD allele and it is thought that the allele might play a role in the development and progression of disease.

There is a tight link between cell cycle control and polyadenylation machinery suggesting an important role of this machinery in the development of cancer. Cyclin-dependent enzymes seem to regulate the activity of the polyadenylation machinery. The amounts of some factors of the mRNA 3' processing machinery (CstF) increase in mitotically active cells in phases of the cell cycle preceding DNA synthesis. The amount of the 64-kDa subunit CstF-64 increases 5-fold during the G0 to S phase transition and concomitant proliferation induced by serum in 3T6 fi-broblasts. The increase in CstF-64 is associated with the G0 to S phase transition. Cdc2-cyclin B phosphorylates PAP at the Ser-Thr-rich region.

However, as it seems now, most diseases associated with defects in mRNA processing are caused by mutations in cis-acting elements that disrupt sequences essential for pre-mRNA splicing. These can be canonical sequences at the intron-exon border or located within an exon. They directly affect the expression of a single mutated gene. Approximately 15% of the nucleotide substitutions that cause human diseases disrupt pre-mRNA splicing. Thus these diseases do not seem to be directly caused by alterations in the polyadenyation/cleavage-machinery.

However, since recently evidence for a number of interrelationships between polyadenylation/cleavage and splicing is accumulating (for review see Zhao, Hyman and Moore in Microbiology and Molecular Biology Reviews, 1999, pp. 405-445), it might very well be that alterations in the 3'-end processing machinery contribute to the etiology of these diseases.

### Examples of diseases caused by incorrect splicing are mentioned below:

Amyotrophic lateral sclerosis (ALS) is a neurodegenerative disease involving degeneration of cortical motor neurons and spinal/bulbar motor neurons. In the sporadic form of the disease, the neuron degeneration is caused by excessive extracellular glutamate. The glutamate transporter functional in the CNS is the astrocyte EAAT2 which is altered in ALS. The pre-mRNA for EAAT2 is aberrantly spliced in the brain regions affected. The reason for this is still unknown, but the defect lies probably in one or a few auxiliary splicing factors that regulate the splicing of a sub-set of pre-mRNA in these cells. The factors have not yet been identified.

The human papillomavirus (HPV) E2 protein plays an important role in transcriptional regulation of viral genes as well as in viral DNA replication. HPV-5 (an EV epidermodysplasia verruciformis-HPV) protein can specifically interact with cellular splicing factors including a set of prototypical SR proteins and two snRNP-associated proteins (Lai, Teh et al. 1999, J. Biol. Chem. 274, pages 11832-41). Interestingly all these three viruses have been associated with cancer progression. Papillomavirus infection precedes cervical cancer, whereas EBV and HSV-8 have been described in association with lymphomas.

In hepatocellular carcinoma, there is a defect in mRNA splicing. In this disease, there are anti-nuclear antibodies to a 64 kD protein, which has splicing factor motifs. A defect in the regulatory subunit 3 of the protein phosphatase 1(PP1) has been found in haematological malignancies and in lung, ovarian, colorectal and gastric cancers. Low PP1 activity has been observed also in acute myelogenous leukaemia.

Heterogeneous nuclear ribonucleoproteins (hnRNP) associate with pre-mRNA and have a role in RNA processing and splice site selection. HnRNP A2 shows a marked overexpression in lung cancer and brain tumours and has thus been used as a biomarker for these tumor types.

The development of antinuclear antibodies (ANA) in malignancies has been described but its mechanism is still not understood. A great diversity of ANA specificities is found in hepatocellular carcinoma. In hepatoma sera antibodies co-localize with non-snRNP splicing factor SC35, suggesting that the antigenic targets might be involved in mRNA splicing. Hepatocellular carcinoma has a significantly higher frequency of ANA than chronic hepatitis C, chronic hepatitis B, alcoholic liver cirrhosis or healthy donors.

In some autoimmune diseases, a possible link has been detected to a preceding virus infection, like Epstein-Barr virus in SLE. Furthermore it seems that even vaccination is potentially dangerous: a candidate for cytomegalovirus CMV vaccine is glycoprotein gB (UL55). Immunization with an adenovirus-gB construct (Ad-gB) not only induces a significant anti-viral response, but a significant IgG auto-antibody response (p > 0.005) to the U1-70 kDa spliceosome protein. Auto-antibodies to U1-70 kDa are part of the anti-ribonucleoprotein response seen in systemic lupus erythematosus and mixed connective tissue disease.

At least two molecules which are also part of the complex are known to be inhibited by natural toxins or treatment against various diseases.

Protein phosphatase 1 is inhibited by several natural product toxins.

The marine toxins include the cyanobacteria-derived cyclic heptapeptide microcystin-LR and the polyether fatty acid okadaic acid from dinoflagellate sources. They bind to a common site on PP1. The dephosphorylation of PP1 is inhibited ( among other serine/threonine phosphatases PP2A, PP2B, PP2C and PP5/T/K/H) by Fumonisin B1 (FB1), a mycotoxin produced by the fungus Fusarium moniliforme. This is a common contaminant of corn, and is suspected to be a cause of human esophageal cancer. FB1 is hepatotoxic and hepatocarcinogenic in rats, although the mechanisms involved have not been clarified.

### Viral proteins are able to interfere with PP1 activity:

The transcription factor EBNA2 of the Epstein-Barr virus induces the expression of LMP1 onco-gene in human B- cells. EBNA2A from an EBV-immortalized B-cell line co-immunopurifies with a PP1-like protein. A PP1-like activity in nuclear extracts from EBV-immortalized B-cell line can be inhibited by a GST-EBNA2A fusion product.

Poly(A)polymerase (PAP) is affected by anticancer drugs and is inhibited by some antiviral agents.

### Anticancer drugs:

Most anticancer drugs act through the mechanism of apoptosis. Apoptosis may be regulated at all levels of gene expression including the addition of the poly(A) tail to the 3' end of mRNAs. Drug combinations are more effective than single drugs and various chemotherapeutic strategies have therefore been developed. Dimethylsulfoxide (DMSO) in combination with interferon (IFN) results in pronounced PAP dephosphorylation, activity reduction and apoptosis of HeLa cells.

Purine and pyrimidine analogues often affect PAP activity. They are potentially useful agents for chemotherapy of cancer diseases. The anticancer drugs 5-Fluorouracil (5-FU), interferon and tamoxifen mediate both partial dephosphorylation and inactivation of poly(A) polymerase (PAP).

PAP (from isolated hepatic nuclei) is inhibited by cordycepin 5'-triphosphate.The nucleoside analogue cordycepin is a therapeutic agent for TdT+ (terminal deoxynucleotidyl transferase positive) leukemia. In the presence of an adenosine deaminase inhibitor, deoxycoformycin (dCF), cordycepin is cytotoxic to leukemic TdT+ cells. A cordycepin analog of (2'-5') oligo(A) which can be synthesized enzymatically from cordycepin 5'-triphosphate and the core cordycepin analog can replace human fibroblast interferon in preventing the transformation of human lymphocytes after infection with Epstein-Barr virus B95-8 (EBV). The core cordycepin analog is not cytotoxic to uninfected lymphocytes and proliferating lymphoblasts.

Not only is PAP affected by anticancer drugs, but it has a possible use as a tumor marker involved in cell commitment and/or induction of apoptosis and could be used to evaluate tumor cell sensitivity to anticancer treatment.

### Antiviral drugs:

Ara-ATP (arabinofuranosyladenosine triphosphate) is an antiherpetic drug that inhibits herpes simplex virus replication. It inhibits poly(A) polymerase activity by competing with ATP. It blocks both cleavage and polyadenylation reactions by interacting with the ATP-binding site on poly(A) polymerase, the activity of which is essential for the cleavage reaction.

Purine and pyrimidine analogues are also used as antiviral agents. As an example, the most extensively used drug against HSV is idoxyuridine, the 5'-amino analog of thymidine.

A decrease in herpes simplex virus transcription and perturbation of RNA polyadenylation is induced by 5'-amino-5'-deoxythymidine (AdThd).

### The cleavage stimulation factor (CSTF):

Treatment with hydroxyurea or ultraviolet light strongly, but transiently, inhibits 3' cleavage. This is accompanied by increased amounts of a CstF/BARD1/BRCA1 complex, though the amount of these proteins remains the same.

Despite the large body of information already available from the prior art concerning the cleavage/polyadenylation machinery of precursor mRNA up to now not all components of the machinery are known not to speak of the composition of the complex as a whole.

An object of the present invention was to identify and provide new components of the cleavage/polyadenylation machinery and to provide new targets for therapy.

Said object is achieved by a component of the cleavage/polyadenylation machinery of precursor mRNA selected from Yeast proteins YGR156W comprising the sequence of SEQ ID NO:2, YKL018w comprising the sequence of SEQ ID NO:3, YOR179c comprising the sequence of SEQ ID NO:4 and YKL059c comprising the sequence of SEQ ID NO:1, the mammalian homologs of these proteins, derivatives of the above proteins carrying one or more amino acid substitutions, deletions and/or additions and proteins showing a percent identity of at least 40% to any of the proteins of a) and/or b).

"Mammalian homologs" as used herein means a protein of the cleavage/polyadenylation machinery of a mammal which performs the same function as the corresponding yeast protein.

Such homologs are also termed "orthologue gene products". The algorithm for the detection of orthologue gene pairs from yeast and human uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given e.g. in Nature, 2001, vol. 409, p. 860-921.

"Derivative" of the protein according to the invention means a protein which is the outcome of a modification of the naturally occurring protein, by amino acid substitutions, deletions and additions, respectively, which derivatives still exhibit the biological function of the naturally occurring protein although not necessarily to the same degree. The biological function of such proteins can e.g. be examined by available in vitro cleavage/polyadenylation assays as will be described below.

"Percent identity" means the number of identical residues as defined by an optimal alignment using the Smith-Waterman algorithm divided by the length of the overlap multiplied by 100. The alignment is performed by the search program (W.R. Pearson (1991) Genomics 11:635-650) with the constraint to align the maximum of both sequences.

"Target for therapeutic drug" means that the respective protein (target) can bind the active ingredient of a pharmaceutical composition and thereby changes its biological activity in response to the drug binding.

"Effector of the cleavage/polyadenylation of precursor mRNA" means a compound that is capable of binding to a member of the cleavage/polyadenylation machinery thereby altering the cleavage/polyadenylation activity of the complex. This altering can be a reduction or increase in cleavage/polyadenylation activity.

The component according to the invention is preferably a protein component which can be further modified e.g. by carbohydrate residues. The components can either be prepared by recombinant DNA technology based on the sequences provided by the present invention or can be isolated from a biological source by using the process according to the invention.

The mammalian homologs or "orthologues" of the yeast proteins according to the invention can either be isolated based on the sequence homology of the yeast genes to the mammalian genes by cloning the respective gene applying conventional technology and expressing the protein from such gene or by isolating the mammalian proteins according to the process of the invention as explained in more detail below.

The derivatives of the proteins according to the invention can be produced e.g. by recombinant DNA technology applying the standard technology to modify the amino acid sequence of a given protein via the modification of the underlying gene using e.g. site directed mutagenesis, etc.

A protein that shows a certain degree of identity to the naturally occurring proteins from mammals and/or yeast, respectively, can also be prepared e.g. by applying recombinant DNA technology as described above for the derivatives according to the invention. Alternatively, such protein can be isolated from natural sources by applying the process of the invention.

By applying the process according to the invention to the isolation of the
polyadenylation/cleavage machinery from yeast seven new proteins could be identified in said yeast complex.

YKL059c: having the amino acid sequence of SEQ ID NO:1 is the product of an essential gene and is a zinc binding protein containing a C2HC Zinc finger. The presence of this domain predicts a RNA binding function of YKL059c. We believe the corresponding gene product is identical to Pfs1, a protein which has been mentioned in several publications, but which has never been annotated in the databases (for review see Keller, W. and Minvielle-Sebastia (1997). Curr Opin Cell Biol 11: 352-357).

YGR156w is the protein product of an essential gene having the sequence according to SEQ ID NO:2. This protein also contains a RNA binding motif.

YKL018w is also an essential protein containing a WD40 domain which is a typical protein binding domain. Said protein has the amino acid sequence according to SEQ ID NO:3.

YOR179c shows significant sequence similarity to Ysh1 and has the sequence according to SEQ ID NO:4.

Ref2 (YDR195w) is a known protein (GenBank Acc. No. CAA88708.1). It is a non-essential gene product. It has been shown to be involved in 3'-end formation prior to the final polyadenylation step. However, Ref2 has never been identified before as a component of the 3'-end processing machinery. Ref2 has been shown to interact with Glc7, another new component of the cleavage/polyadenylation machinery.

Glc7 (YER133w) is also a known protein (GenBank Acc. No. AAC03231.1). It is also an essential protein and is a Type I protein serine threonine phosphatase which has been implicated in distinct cellular roles, such as carbohydrate metabolism, meiosis, mitosis and cell polarity. Its occurrence in the cleavage/polyadenylation machinery has not been known before.

Ssu72 (YNL222w) is also a known protein (GenBank Acc. No. CAA96125.1) and is an essential phylogenetically conserved protein which has been shown to interact with the general transcription factor TFIIB (Sua7). TFIIB is an essential component of the RNA polymerase II (RNAP II) core transcriptional machinery. It is thought that this interaction plays a role in the mechanism of start site selection by RNAP II. The finding according to the present invention that Ssu72 is associated with Pta1 is likely to be relevant since it is believed that mRNA 3'-end formation is linked with other nuclear processes like transcription, capping and splicing. Furthermore, Ssu 72 has also been clearly identified in a "reverse tagging experiment" as explained herein below by using some of the Pta1 associated proteins as bait. However, when Ssu72 itself was used as a bait associated proteins were not found most likely due to the fact that the addition of a C-terminal tag renders Ssu72 non-functional.

In a preferred embodiment the proteins show at least 40%, more preferably 70%, most preferably 90% identity to the yeast proteins or their mammalian homologs.

In a further preferred embodiment the proteins are encoded by a nucleic acid which hybridizes to a nucleic acid encoding a protein according to any of SEQ ID Nos: 1 to 4.

The present invention further relates to a fusion protein comprising a component according to the invention. The fusion part, which can be added to the N-terminal, the C-terminal or into the amino acid sequence of the component according to the invention may comprise a few amino acids only e.g. at least five, which amino acids for example provide an epitope which is then be used as a target for affinity purification of the protein and the complex, respectively. Such a type of added amino acid is also termed "tag" throughout the present specification (optionally, the fusion protein may comprise even more than one such fusion partner).

The present invention further relates to a new protein complex which is useful for cleaving and/or polyadenylating a nucleic acid and which complex comprises at least one of the components according to the invention. Such a complex can be isolated from a natural source by applying the process according to the invention or can be reconstituted from the different components made available by the present invention.

In a preferred form the complex comprises at least proteins YGR156w, YKL018w, YOR179c and YKL059c and most preferably also contains the further proteins Ref2, Glc7 and Ssu72. A summary of the known components and the new components according to the invention as can be found in the mRNA 3'-end processing machinery is shown in Table 1.

The present invention also relates to a nucleic acid encoding a component or fusion protein according to the invention. Preferably, the nucleic acid is selected from those encoding any of the proteins YGR156w, YOR179c, YKL018w and YKL059c or the nucleic acid hybridizing thereto and coding for a protein with similar biological function.

The present invention further relates to a construct which comprises the nucleic acid according to the invention and at least one further nucleic acid which is normally not associated with the nucleic acid according to the invention. Such a construct is preferably a vector which preferably is capable of replicating in a given cell and contains the necessary transcription control elements for expressing the nucleic acid according to the invention in a given expression system. Moreover, such vector construct may contain selection markers.

The present invention also relates to a host cell containing a nucleic acid according to the invention or a construct according to the invention. Such a host cell may contain an expression vector which encodes a component according to the invention which component may serve as a bait in order to isolate the further proteins of the complex and which at least partly interact with the bait. Host cells can be prokaryotic and eukaryotic cells, whereas mammalian host cells are preferred.

The present invention further relates to the use of the products according to the invention in therapy wherein the products according to the invention are useful as a target for a therapeutic drug. It is known from the prior art that mRNA 3'-end processing is involved in viral growth, in the development of cancer and in certain neurodegenerative diseases. By having identified new components of the cleavage/polyadenylation machinery the present invention, hence, offers new targets for treating viral diseases, cancer and neurodegenerative diseases. By affecting the biological activity of the components of the invention and/or by affecting the complex as a whole the cleavage/polyadenylation activity thereof can be influenced depending by the needs of the patient to be treated.

The present invention further relates to a process for preparing a component of claim 1 comprising the following steps:
tagging a protein of the mRNA 3' end processing complex (cleavage/polyadenylation machinery) with a sequence that allows affinity purification of the tagged protein,
expressing such protein in a target cell,
isolating the protein complex which is attached to the tagged protein,
disassociating the protein complex and isolating the individual complex members.

The present invention further relates to a pharmaceutical composition comprising a product according to the invention. Such pharmaceutical composition contains beside the product according to the invention as active ingredient further excipients and additives as known by a skilled person.

The present invention further relates to a kit for processing RNA which kit comprises a product according to the invention. Such a kit may contain e.g. expression vectors encoding the essential components of the cleavage/polyadenylation machinery which components after being expressed can be reconstituted in order to form a biologically active cleavage/polyadenylation complex. Such a kit preferably also contains the required buffers and reagents together with the working instructions.

The present invention further relates to a kit for the diagnosis of diseases of mammals which kit comprises a product according to the invention. As stated above the polyadenylation/cleavage machinery is involved in a large number of diseases. If said machinery activity is changed due to e.g. mutations of some components thereof and/or effectors, this may have severe implications on the affected organism. The kit according to the present invention containing the products according to the invention allows to examine as to whether the cleavage/polyadenylation machinery in a given sample might show some defects. Such a kit may be used to determine genetic defects of the genes encoding the components of the cleavage/polyadenylation machinery.

The present invention further relates to an antibody which reacts with a product according to the invention. Such an antibody might be used e.g. during purification of the machinery from a given source by affinity purification methods. Moreover, the antibody might be used in diagnosis in order to detect changes and/or modifications of a product according to the invention in a given sample.

The present invention further relates to a process for the identification and/or preparation of an effector of the cleavage/polyadenylation of precursor mRNA comprising the step of bringing into contact a product of any of claims 1 to 7 with a compound, a mixture or a library of compounds and determining whether the compound or a certain compound of the mixture or library binds to the product and/or effects the products biological activity and optionally further purifying the compound positively tested as effector. Binding assays for determining as to whether a given compound interacts with a certain protein are well established in the art. Moreover, a number of polyadenylation assays are described in the prior art. Such assays are for example disclosed in Butler, S. J. and Platt, T. (1988), Science 242, 1270-1274; Kessler, M.M.; Zhao, J. and Moore, C.L. (1996), J. Biol. Chem. 271, 27167-27175; Bienroth, S.E.; Wahle, C.; Suter-Crazzolara, C. and Keller, W. (1991), J. Biol. Chem. 266, 19768-19776; Edwards-Gilbert, G. and Milcarek, C. (1995), Mol. Cell. Biol. 15, 6420-6429; Moore, C.L. and Sharp, P.A. (1985), Cell 41, 845-855,; Wahle, E. (1991), Cell 66, 759-768; Christofori, G. and Keller, W. (Cell) 54, 875-889. A specific assay for the cleavage step which is involved with polyadenylation has been disclosed in Ruegsegger, U., Beyer, K. and Keller, W. (1996), J. Biol. Chem. 271, 6107-6113.

The present invention, hence, allows the identification of new effectors which affect the biological activity of the cleavage/polyadenylation machinery of precursor RNA. Said effectors than can be used to modify the cleavage/polyadenylation machinery in a cell by introducing an effector into a cell. Moreover, the mRNA processing activity of a given cell can also be affected by introducing a product according to the invention into such cell.

Moreover, the products according to the invention are very useful for identifying new effectors of cleavage/polyadenylation of precursor mRNA.

Fig. 1 shows a schematic presentation of motifs underlying polyadenylation in mammals and yeast.

Fig. 2 shows the protein pattern as obtained by the separation of the members of the cleavage/polyadenylation complex of yeast using pta1 as a bait using tap.

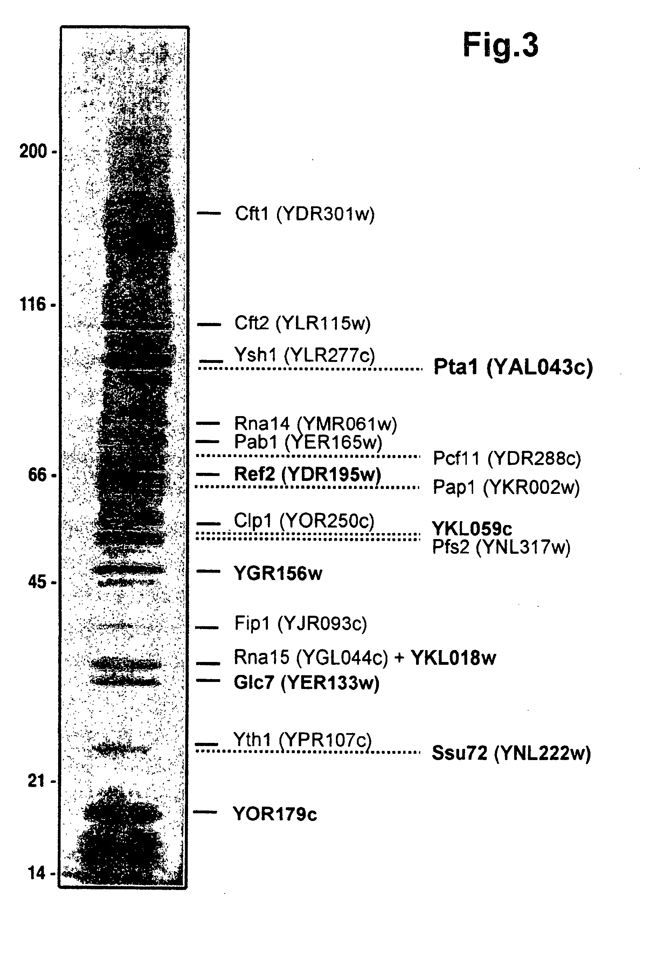

Fig. 3 shows the same pattern as Fig. 2 but the new members of the polyadenylation/cleavage complex are indicated in bold letters.

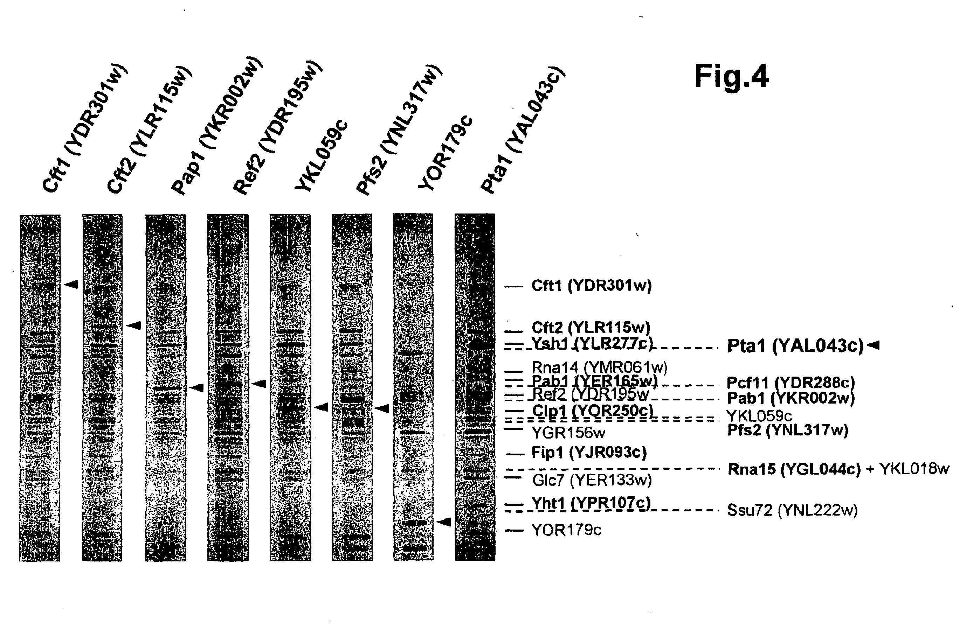

Fig. 4 shows the protein pattern obtained by the reverse tagging and re-purification. The analysis of the bands clearly identifies all seven novel interactors (Ref2, YKL059c, YGR156w, YKL018w, YOR179c, Glc7 and Ssu72) These data does provide biological validation that all of the seven proteins are bonafide components of a 3'-end processing machinery.

The following examples illustrate the invention.

### CONSTRUCTION OF A YEAST STRAIN EXPRESSING TAP-TAGGED Pta1 (see also table 4)

### PURIFICATION OF PROTEINS ASSOCIATED WITH PTA1

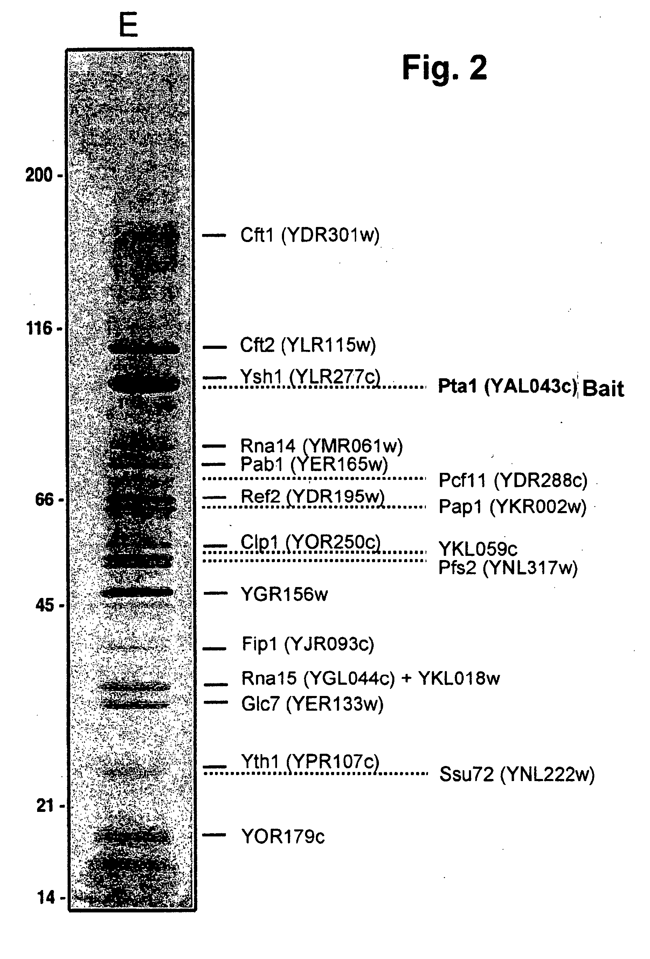

The TAP-technology, which is more fully described in WO/0009716 and in Rigaut, G. et. al. (1999), Nature Biotechnology. Vol. 17 (10): 1030-1032 respectively was used for protein complex purification. The Pta1 protein was C-terminally tagged with a TAP-tag which consists of calmodulin-binding peptide (CBP), a cleavage site for TEV protease followed by two IgG-binding units of protein A (Rigaut, G. et. al. (1999), Nature Biotechnology. Vol. 17 (10): 1030-1032). Pta1 is an essential protein which has been reported to be a component of PFI. Pta1-TAP was used as a bait to identify associated partners from cell lysates using the two-step TAP purification procedure. Proteins were separated by 1 D gel electrophoresis and visualized by staining with Coomassie. More than a total of 20 bands could be detected on the gel (see Fig. 2). The identity of the proteins was determined by mass spectrometry. 13 of these are known components of the pre-mRNA processing machinery: Cft1, Cft2, Ysh, Pta1, Rna14, Pab1, Pcf11, Pap1, Clp1, Pfs2, Fip1, Rna15 and Yth1. It is to be noted that such a comprehensive number has never before been purified together in form of a complex. The remaining seven proteins (see fig. 2, highlighted proteins) have not previously been found associated with Pta1: Ref2, YK059c, YGR156w, YKL018w, Glc7, Ssu72 and YOR179c.

### VALIDATION OF INTERACTIONS FOUND WITH Pta1

A reciprocal experiment to the one described above was performed. For this purpose a subset of the interactors found in the above described Pta purification (both known and novel interactors) were chosen as a bait for a further round of purification. In the case of some proteins the C-terminally tagged versions could not be recovered. The likely reason for this is that the addition of the TAP tag at the C-terminus interferes with the function of these proteins. An important fact is that almost all of the known components involved in 3'-end formation and five of the seven novel proteins identified herein are essential for cell viability.

### SEQUENCE ANALYSIS OF MEMBERS OF THE COMPLEX

The process of mRNA processing is highly conserved in eukaryots. Accordingly, for 15 of the yeast proteins human orthologues could be found. This illustrates that many of the functions found in the yeast complex can be transferred to humans. Also the enzymatic activity of this complex has long been known, the enzymatically active member could not yet be unraveled. Using extensive sequence similarity searches it could be shown that Ysh1 is homologous to a class of bacterial beta-lactamases. The active center of this protein family contains 2 zinc ions which are bound by histidines. As these residues are conserved in Ysh1 and it was shown that enzymatic activity of the yeast complex is zinc dependent predicted that Ysh1 is responsible for the catalytic activity of the complex. Two other proteins found in the complex, Cft2 and YOR179c, are homologous to the Ysh1 N- and C-terminus, respectively. Though Cft2 is homologous to the enzymatic region of Ysh1 it misses the zinc binding histidines indicating that it lacks enzymatic activity. Thus, Cft 2 and YOR179c could compete with Ysh1 for the same binding slot of the complex, suggesting a novel type of regulation of polyadenylation. A similar way of regulation might be used in the case of Pfs2 and YKL018w, which both consist of multiple WD40 domains.

### PREDICTION OF MAMMALIAN PROTEINS

To allow the transfer of function information from yeast to human proteins, we did not only use an identity cutoff, but also the 'orthology' concept. Orthology defines genes which arose via a speciation event, in contrast to genes which arose via gene duplication. Orthologue genes are supposed to perform the same function in different organisms, therefore more detailed function information can be transferred. The algorithm for the detection of orthologous gene pairs from yeast and human uses the whole genome of these organisms. First, pairwise best hits were retrieved, using a full Smith-Waterman alignment of predicted proteins. To further improve reli-ability, these pairs were clustered with pairwise best hits involving Drosophila melanogaster and Caenorhabditis elegans proteins. See "Initial sequencing and analysis of the human genome", Nature 2001 Feb 15; 409(6822):860-921 for a detailed description of the analysis.

### Bioinformatic analysis of the Complex:

Functional domains of all members of the complex were analyzed using SMART (SMART: a web-based tool for the study of genetically mobile domains. Nucleic Acids Res 2000 Jan 1; 28(1):231-4) and Pfam (Pfam: protein families database, Nucleic Acids Res 2000 Jan 1; 28(1):263-6).

### COMPARISON OF THE YEAST AND MAMMALIAN CLEAVAGE/POLYADENYLATION MACHINERY

The sequence of many of the polypeptides involved in 3'-end formation are conserved form yeast to mammals, although the sequence elements on the substrate pre-mRNA differ (see table 3).

### PURIFICATION OF PROTEIN COMPLEXES FROM YEAST USING Pta1 AS BAIT

Yeast strain Sc0051 expressing TAP-tagged Pta1 was cultured in 4 I of YPD medium to an OD600 of 2. After harvesting, the cell pellet was frozen in liquid nitrogen and stored at -80°C. All further manipulations were done at 4°C except where noted. For preparation of protein lysates the cells were resuspended in lysis buffer (50 mM Tris-HCI pH 7.5, 100 mM NaCI, 0.15 % NP-40, 1.5 mM MgCl2, 0.5 mM DTT, protease inhibitors) and subjected to mechanical disruption with glass beads. Lysates were clarified by two successive centrifugation steps at 20.000 x g for 10 min and 100.000 x g for 1 hour. After addition of glycerol to 5 % final concentration the lysates were frozen in liquid nitrogen and stored at -80°C.

For the first purification step 500 µl of rabbit IgG-Agarose (50:50 slurry, Sigma A2909) pre-equilibrated in lysis buffer were added to the lysate and the sample was rotated for 2 hours. The unbound fraction was discarded and the beads with the bound material were transferred to a 0.8 ml column (MoBiTec M1002, 90 µm filter). The beads were washed with 10 ml of lysis buffer followed by 5 ml of TEV cleavage buffer (10 mM Tris-HCI pH 8.0, 100 mM NaCI, 0.1 % NP-40, 0.5 mM EDTA, 1 mM DTT).

150 µl of TEV cleavage buffer and 4 µl of TEV protease were added to the column and the sample was incubated on a shaker at 16 °C for 2 hours. The eluate was recovered by pressing with a syringe.

150 µl of Calmodulin dilution buffer (10 mM Tris-HCI pH 8.0, 100 mM NaCI, 0.1 % NP-40, 2 mM MgAc, 2 mM imidazole, 4 mM CaCl2, 1 mM DTT) was added to the previous eluate and this mixture was transferred to a MoBiTec column containing 300 µl (bead volume) of Calmodulin affinity resin (Stratagene #214303) which was prewashed in Calmodulin wash buffer (10 mM Tris-HCI pH 8.0, 100 mM NaCI, 0.1 % NP-40, 1 mM MgAc, 1 mM imidazole, 2 mM CaCl2, 1 mM DTT). The samples were rotated for 1 hour at 4 °C.

After washing of the beads with 10 ml of Calmodulin wash buffer, protein complexes were eluted with 600 µl of elution buffer (10 mM Tris-HCI pH 8.0, 5 mM EDTA). The samples were concentrated in siliconised tubes in a speed vac to a final volume of 10-20 µl. Proteins were detected by polyacrylamide gel electrophoresis followed by staining with colloidal Coomassie blue.

### PURIFICATION OF PROTEIN COMPLEXES FROM MAMMALIAN CELLS

### Standard Lysis Protocol

The medium was removed from the culture dish and the cells were scraped directly from the plate with help of a rubber policeman. The cells were collected on ice washed 3 times with PBS and resuspended in lysis buffer (50 mM Tris, pH: 7.5; 5 % glycerol; 0,2 % IGEPAL; 1.5 mM MgCl2; 1 mM DTT; 100 mM NaCI; 50 mM NaF; 1 mM Na3VO4 + protease inhibitors). The cells were lysed for 30 min on ice, spun for 10 min. at 20,000g and re-spun for 1h at 100,000g. The supernatant was recovered, rapidly frozen in liquid nitrogen and stored at -80 °C. For pre-clearing the thawed lysate was incubated with 500 µl sepharose CL-4B beads (Amersham Pharmacia) for 1 h shaking and finally processed according the TAP protocol.

### Nuclear Lysis Protocol

The medium was removed from the culture dish and the cells were scraped directly from the plate with help of a rubber policeman. The cells were collected on ice washed 3 times with PBS and resuspended in buffer A (10 mM Tris-CI, pH 7.5; 1, 5 mM MgCl2; 10 mM KCI; 1 mM DTT, 50 mM NaF; 1 mM Na3VO4). To isolate the nuclei the lysate was dounced with a tight fitted pestle in a dounce homogenizer for 15 strokes. The nuclei were harvested by centrifugation (10 min. at 2000 g and 20 min. at 16,000 g) and lysed in buffer B (50 mM Tris-Cl, pH: 7.5; 1.5 mM MgCI; 20 % glycerol; 420 mM NaCI; 1mM DTT; 50 mM NaF; 1 mM Na3VO4) for 30 min. on ice with frequent shaking. The protein lysate was cleared by centrifugation (30 min at 100,000 g) and 1 : 4 diluted with buffer C (50 mM Tris-CI, pH: 7.5; 1 mM DTT; 0.26 % NP40; 1.5 mM MgCI; 50 mM NaF; 1 mM Na3VO4). After 30 min incubation on ice the lysate was re-spun for 30 min at 100,000 g, quickly frozen in liquid nitrogen and stored at -80 °C. For pre-clearing the thawed lysate was incubated with 500 µl sepharose CL-4B beads (Amersham Pharmacia) for 1 h shaking and finally processed according the TAP protocol.

### MASS SPECTROMETRIC ANALYSIS

Protein digestion prior to mass spectrometric analysis: Gel-separated proteins were reduced, alkylated and digested in gel essentially following the procedure described by Shevchenko et al. (Shevchenko, A., Wilm, M., Vorm, O., Mann, M. Anal Chem 1996, 68, 850-858). Briefly, gel-separated proteins were excised from the gel using a clean scalpel, reduced using 10 mM DTT (in 5mM ammonium bicarbonate, 54 °C, 45 min) and subsequently alkylated with 55 mM iodoacetamid (in 5 mM ammonium bicarbonate) at room temperature in the dark (30 min). Reduced and alkylated proteins were digested in gel with porcine trypsin (Promega) at a protease concentration of 12.5 ng/µl in 5mM ammonium bicarbonate. Digestion was allowed to proceed for 4 hours at 37 °C and the reaction was subsequently stopped using 2 µl 25% TFA.

### Desalting and concentration of peptides produced by in-gel digestion of gel-separated proteins:

Peptides were desalted and concentrated using a prefabricated uZipTip (Millipore) reversed phase column. Peptides were eluted directly onto stainless steel MS sample holders using 2µl eluent (70% acetonitrile in 5% TFA containing 2mg/ml alpha-Cyano-4-hydroxy-cinnamic acid and two standard peptides for internal calibration of mass spectra).

### Mass spectrometric data acquisition:

Matrix-assisted laser desorption/ionisation (MALDI) time-of-flight (TOF) mass spectra were acquired in delayed extraction mode on a Voyager DE-STR PRO MALDI mass spectrometer (Applied Biosystems) equipped with a 337 nm nitrogen laser. 500 laser shots were averaged in order to produce final spectra. Spectra were automatically internally calibrated using two standard peptides. The monoisotopic masses for all peptide ion signals detected in the acquired spectra were determined and used for database searching.

### Protein sequence database searching using peptide mass fingerprinting (PMF) data:

The list of monoisotopic peptide masses obtained from the MALDI mass spectrum was used to query a fasta formatted protein sequence database that contained all protein sequences from S. cerevesia. Proteins were identified by peptide mass fingerprinting (Mann, M., Højrup, P., Roep-storff, P. Biol Mass Spectrom 1993, 22, 338-345; Pappin, D., H¿jrup, P, Bleasby, AJ Curr. Biol. 1993, 3, 327-33; Henzel, W. J., Billeci, T. M., Stults, J. T., Wong, S. C., Grimley, C., Watanabe, C. Proc Natl Acad Sci USA 1993, 90, 5011-5015; Yates, J. R., Speicher, S., Griffin, P. R., Hunkapiller, T. Anal Biochem 1993, 214, 397-408; James, P., Quadroni, M., Carafoli, E., Gonnet, G. Biochem Biophys Res Commun 1993, 195, 58-64) using the software tool Profound (Proteometrics). In PMF, a protein is identified by correlating the measured peptide masses with theoretical digests of all proteins present in the database. Search criteria included: tryptic protein cleavage, monoisotopic masses, 30 ppm mass accuracy. No restrictions on protein size or isoelectric point were imposed.

**TABLE 1**

| Known components of the yeast mRNA 3'-end-processing machinery | | | | |
|---|---|---|---|---|
| Factor | Function | Polypeptide composition (kDa) | Gene product / (ORF) | Sequence motifs |
| CF IA | Cleavage and | 79.8 | Rna14 (YMR061w) | 8xHAT domains |
| | polyadenylatio | 71.9 | Pcf11 (YDR228c) | |
| | n | 50.0 | Clp1 (YOR250c) | |
| | | 32.8 | Rna15 (YGL044c) | RRM |
| | | 64.2 | Pab1 (YER165w) | 4xRRM 1xPolyA |
| | Polyadenylatio | | | |
| | n | | | |
| CF IB | Cleavage site | 73 | Hrp1 (YOL123w) | |
| | selection and | | | |
| | polyadenylatio | | | |
| | n | | | |
| CF II/PF I | Cleavage and | 153.4 | Cft1/Yhh1 (YDR301w) | --- |
| (= CPF) | polyadenylatio | 96.1 | Cft2/Ydh1 (YLR115w) | --- |
| | n | 87.6 | Ysh1/Brr5 (YLR277c) | lactamase |
| | | 88.3 | Pta1 (YAL043c) | --- |
| | | 64.4 | Pap1 (YKR002w) | NTP_transfer_2 |
| | | | Pfs1 | |
| | | 53.1 | Pfs2 (YNL317w) | 7xWD40 |
| | | 35.6 | Fip1 (YJR093c) | --- |
| | | 24.4 | Yth1 (YPR107c) | 5xZnF_C3H1 |

| Novel complex members found by CellZome | | | | |
|---|---|---|---|---|
| | | 59.7 | Ref2 (YDR195w) | --- |
| | | 59.5 | YKL059_{c} = Pfs1 | ZnF_C2HC |
| | | 46.8 | YGR156w | RRM |
| | | 37.0 | YKL018w | 4xWD40 |
| | | 35.9 | Glc7(YER133w) | PP2Ac |
| | | 23.3 | Ssu72 (YNL222w) | --- |
| | | 20.9 | YOR179c | similarity to Ysh1 |

| | | | | |
|---|---|---|---|---|
| CF: cleavage factor | | | | |
| PF I: polyadenylation factor CstF: cleavage and stimulation factor CPSF: cleavage and polyadenylation specificity factor | | | | |
| YGR156w: has RNA-binding domain | | | | |
| Glc7: was found in Y2H using Ref2 as bait (Uetz screen) | | | | |
| YOR179c: similar to Ysh1 (PF I complex) (37% identity, 56% similarity) | | | | |

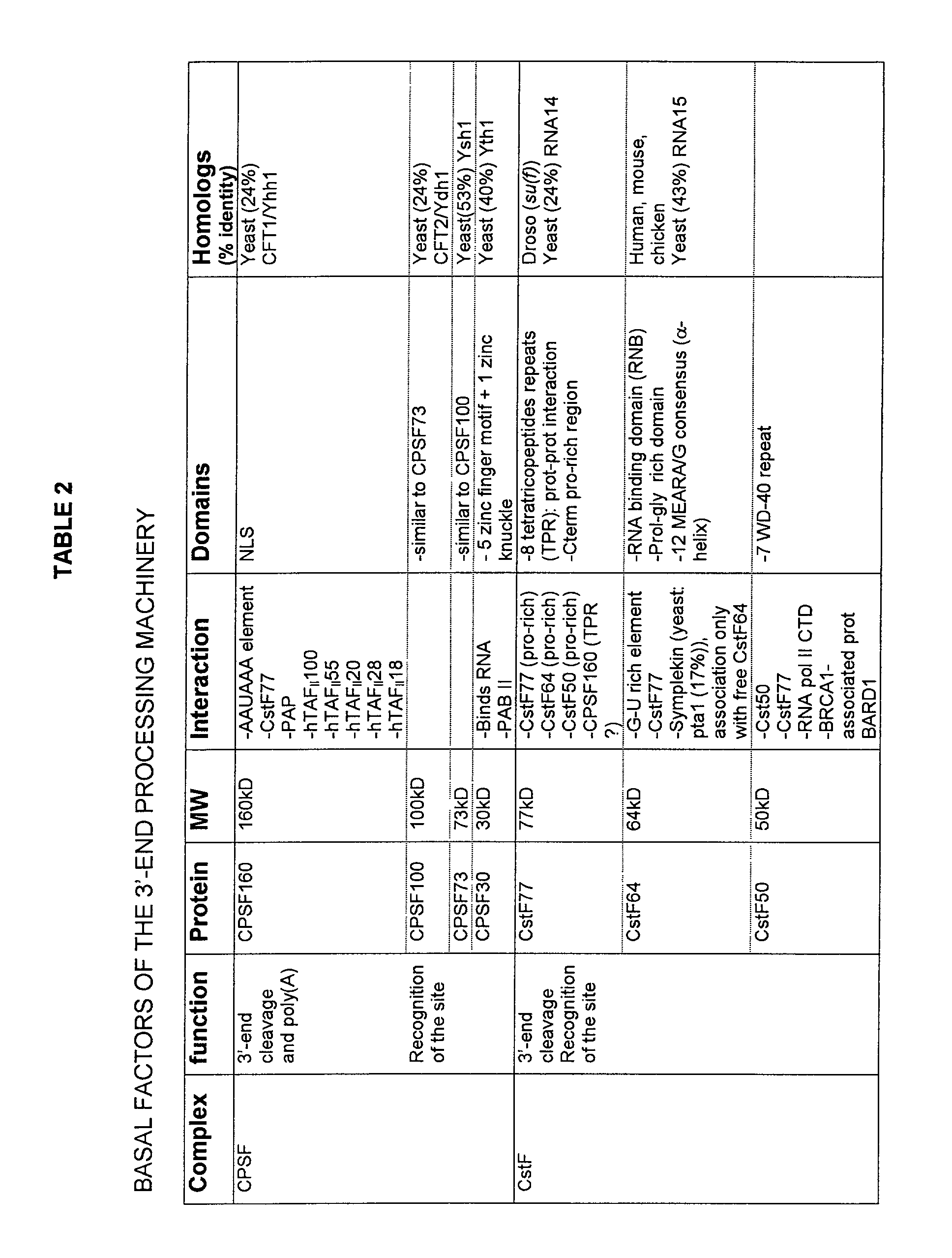

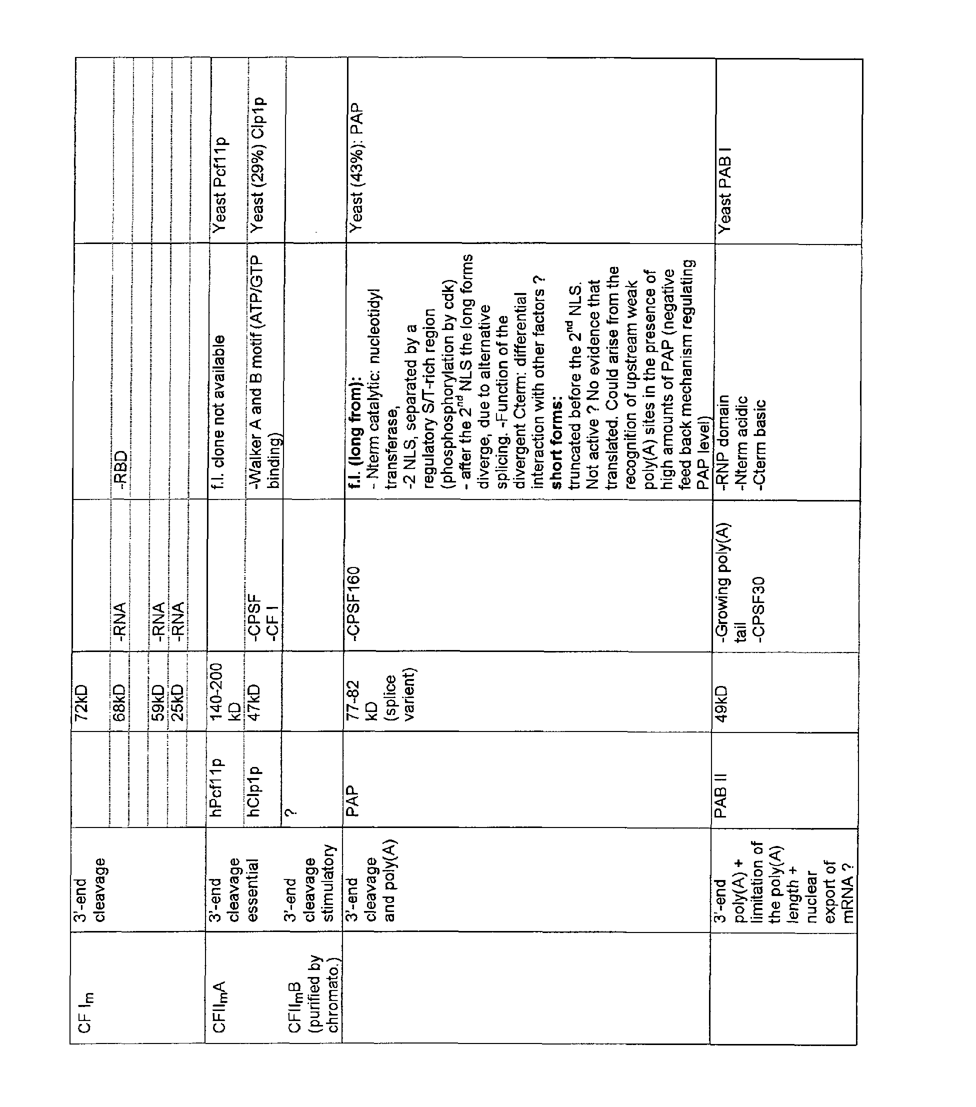

| **Mammalian** | | **Yeast** | |
|---|---|---|---|
| **complex/function** | **Protein/function** | **Protein/function** | **complex/function** |
| CPSF, cleavage+polyadenylation | CPSF160, binding AAUAAA, link with transcription, binds CstF | Yhh1 (CFT1), | PF I, polyadenylation |
| CPSF, cleavage+polyadenylation | CPSF100, | Ydh1 (CFT2) | PF I, polyadenylation |
| CPSF, cleavage+polyadenylation | CPSF73 | Ysh1 | PF I, polyadenylation |
| CPSF, cleavage+polyadenylation | CPSF30, binds mRNA, PABII | Yfh1 | PF I, polyadenylation |
| CstF, cleavage | CstF77, binds all other CstF subunits | RNA14 | CF1, cleavage and polyadenylation |
| CstF, cleavage | CstF64, binds G-U rich | RNA15 | CF1, cleavage and polyadenylation |
| CstF, cleavage | CstF50, binds RNA pol II | | |
| CstF, cleavage | Symplekin, binds CstF64 | Pta1 | PF I, polyadenylation |
| CF Im, cleavage | CF Im 72 | | |
| CF Im, cleavage | CF Im 68 | | |
| CF Im, cleavage | CF Im 59 | | |
| CF Im, cleavage | CF Im 25 | | |
| CF II, cleavage | hPcf11p | Pcf11p | CF1, cleavage and polyadenylation |
| CF II, cleavage | HClp1p, binds CPSF and CF I | Clp1p | CF1, cleavage and polyadenylation |
| cleavage+polyadenylation- | PAP, poly(A) polymerase | PAP | PF I, polyadenylation |
| polyadenylation | PAB II, binds poly(A), nuclear export ? | PAB I | CF1, cleavage and polyadenylation |
| | | Pfs1 | PF I, polyadenylation |
| | | Psf2 | PF I, polyadenylation |
| | | Fip1 | PF I, polyadenylation |

### 1) Oligo design

The oligo are semi-automatically designed in FileMaker Pro. The 3' end of the ORF (forward oligo, 51 nucleotides), the 3' UTR (100 nucleotides used to design the reverse oligo) and sequence 500 nucleotides upstream of the stop codon (40 nucleotides, check oligos) are directly extracted from database (SDG). For the reverse oligo, the program automatically gives the antiparallel sequence. The 17 constant nucleotide sequences used to prime the PCR reaction are automatically added at the 3' end of the oligo.

### 2) Target PCR

### fully automated (Tecan robot)

Materials: target plasmid: pBS1539 (described in Rigaut G, et al., Nat Biotechnol. 1999 Oct;17(10):1030-2.)□ oligos: purchased from MWG, forwards and reverse primers are pre-mixed to a final concentration of 10 micromolar and delivered in a 96 well plate format

| **step** | **volume** | **temperature** | **device** |
|---|---|---|---|
| prepare master mix (AmpliTaq, Perkin Elmer), containing the TAP cassette vector (1.5 ng/25 microliter reaction) | up to 2.5 ml | 4C master mix | cooling platform 1 |
| Dispense the master mix in 96 well plate = **reaction plate** (96 well plate) | 23.5 microliters | 4C master mix + reaction plate | cooling platforms 1 + 2 |
| Add 1.0 microliters of oligo (final concentration: 0.4 microM) to **reaction plate** | 1.0 microliters | 4C reaction plate | cooling platform 2 |
| | | | |
| Cycle (30 cycles) the PCR reaction | | | MJ thermocyclers |
| | | | |
| Pipette 2 microliters from the **reaction plate** to a 96 well gel (Pharmacia) | 2 microliters | rt | Pharmacia ready to run gels |
| run gel 5 minutes | | | Pharmacia, Ready-To-Run |

### 3) Yeast transformation

General considerations: Procedure partially automated Materials: the haploid yeast strain is MGD453-13D: MATa, ade2, arg4, leu2-3,112, trp1-289,□ura3-52.

| **step** | **volume** | **temperature** | **device** | "novelties" verus original protocole. |
|---|---|---|---|---|
| The day before, inoculate 200ml of YPD with ^{~}100 microliters of freshly growing yeast culture | 200 ml | 30C | shaking incubator | |
| When the culture reaches an OD600 of 1.0, dispense 2 ml of yeast culture in 24 well plate = transformation plate (4) | 2ml | rt | | uses of 24 well plates (Qiagen) |
| spin | | rt | heraus centrifuge | |
| | | | | |
| aspirate the sup | 2 ml | rt | | |
| ressuspend cells in the remaining of media | | rt | shaker | no washing steps of the cells |
| dispense 5 microliters of carrier DNA, (Herring testes Carrier DNA; 10 mg/ml; Clontech) | 5 microliters | rt | | |
| mix | | rt | shaker | |
| transfer remaining of PCR from reaction plate to the transformation plate | 20 microliters | rt | | no cleaning of the PCR reaction |
| mix | | rt | shaker | |
| add 500 microliters PEG/LiAc | 500 microliters (viscous) | rt | | |
| mix | | rt | shaker | |
| add 55 microliters DMSO, mix | 50 microliters | rt | shaker | |
| incubate 15' rt | | rt | | |
| incubate 15' at 42C | | 42C | heating platform with custom made Pelletier elements | |
| transfer at rt + 700 microliters TE | 700 microliters | rt | | |
| spin | | rt | | |
| aspirate sup | | rt | | |
| add 800 microliters YPD | 800 microliters | rt | | |
| incubate 30' at 30 C+shake | | 30 C | heating platform + shake r | |
| add 1ml of TE | | rt | | |
| spin | | rt | | |
| aspirate sup | | rt | | |
| add 50 microliters TE | 50 microliters | rt | 8 tips | |
| mix | | rt | shaker | |
| plate+ incubate for ^{~}3 days at 30C (10cm Petri dishes or 12-24 wellplates) | | | | |

### 4) Check PCR

general considerations: fully automated. According to results of the transformation 0 to 6 colonies are tested for homologous recombination. These results are filed in an excell file directly linked to the robot program.
Material: the forward oligos are specific for each ORF (for te sequence cf 1); purchased from MWG at 10 micromolar in 96 well plates. The reverse oligo is constant for all ORFs and annealed in the TAP sequense

| | | | |
|---|---|---|---|
| dispense 20 microliters NaOH (20mM) in 96 well plates | 20 microliters | rt | |
| pickup colonies (0 to 6) in the 96 well plate containing the NaOH = DNA plate (96 well) | | | |
| Boil 2' | | | |
| add 140 microliters of TE pH 7.5 (8x dilution) | | | |
| spin at 2000 rpm | | | |
| Prepare master mix (PCR master, Roche) | up to 9 ml microliters | 4C | cooling platform 1 |
| dispense 14.5 microliters in reaction plate (96 well plate) | 14.5 microliters | 4C | cooling platform 2 |
| add 2.5 microliters of oligo (final concentration 1 microM) to reaction plate | 2.5 microliters | 4C reaction plate | cooling platform 1 |
| transfer 8 microliters DNA from DNA plate to reaction plate | 8 microliters | 4C reaction plate | cooling platform 1 |
| | | | |
| Cycle (30 cycles) the PCR reaction | | | MJ thermocyclers |
| | | | |
| add 1 microliters of LB | 5 microliters | rt | |
| load 10 microliters on a 96 well agarose gel | 10 microliters | | Pharmacia ready to run gels |
| run gel 5 minutes | | | Pharmacia, Ready-To-Run |

### 5) Dot blot analysis

| | | | |
|---|---|---|---|
| the next day, the restreack is used to inoculate 2ml of YPD in 24 well plates (Qiagen). Plates are incubated over night at 30C | | | 30C shaker |
| Culture plates are spin | | | |
| remove the supernatant | | | |
| add 100 microliters of waters | | | mix shaker |
| add 100 microliters of 0.2M NaOH | | | mix shaker |
| incubate 3 minutes at room temperature | | | |
| spin | | | |
| remove the supernatant | | | |
| add 50 microliters of 2x sample buffer | | | |
| boil 3 minutes | | | |
| spin | | | |
| load on a 96 dot blot apparatus | | | Biodot, BioRad |
| dot blot on a nitrocellulose membranne | | | Protran, Schleicher and Schuell |
| detect TAP tagged protein by ECL using peroxidase anti-peroxidase complex | | | |

SEQUENCE LISTING

Amino acid sequences of novel interactors of mRNA 3'-end processing
complex
Ref2 (YDR195w): GenBank # CAA88708.1
1 msapvpqlvn ishalqasti qqirldmvdf nkdcklssiq laridkyids lqaalnqftk
61 dnlhierkek nvteadiqly sglksmyldy lnqliklkhe kqhhstppia ndvsldffvn
121 qlpkfspeer knyidnliln knshnrlskm dglvdavinl cvldtsvaen vrsymklldt
181 lgfqkgsnst gtkanlkkkl asskakikds ekekekekdk skvkmktklk pspllnnddk
241 nsspsptast ssmkklksgl fnkneakste slptsskkkl sfskylnkdd admtklgtkr
301 sidvdfkvnp eastvasnii ssstsgsstt tvatpassee plkkktkisv qdsnvqsilr
361 ngkpkkaris sikflddsql ikvygddlpn qglqvsptql kkilkpfkeg epkeiilfed
421 msiklkpldl mflkntnsdd ymdisetkgg pihcetrtpl iyrknfnhfn pdlnkrppre
481 piefdlngnt nstptiakaf gknslllrkd rgglpykhvp ivkrnkyppr pvh

YKL059c: GenBank # CAA81896.1

1 msstifyrfk sqrntsrilf dgtgltvfdl kreiiqenkl gdgtdfqlki ynpdteeeyd
61 ddafviprst svivkrspai ksfsvhsrlk gnvgaaalgn atryvtgrpr vlqkrqhtat
121 ttanvsgtte eeriasmfat qenqweqtqe emsaatpvff ksqtnknsaq
enegppppgy
181 mcyrcggrdh wikncptnsd pnfegkrirr ttgipkkflk sieidpetmt
peemaqrkim
241 itdegkfvvq vedkqswedy qrkrenrqid gdetiwrkgh fkdlpddlkc
pltggllrqp
301 vktskccnid fskealenal vesdfvcpnc etrdilldsl vpdqdkekev etflkkqeel
361 hgsskdgnqp etkkmklmdp tgtaglnnnt slptsvnngg tpvppvplpf
gippfpmfpm
421 pfmpptatit nphqadaspk k

YGR156w: GenBank # CAA97170.1
1 mtdprrrtgr hfltpenlss tlqitnlppe wnqdiitsvv agsgpvidik akndprtgkl
61 tgvlfdylts kdckraweil nrienfpvki eqiippnykd hlretankns qkqvlqlnrd
121 sypfeaglel pfemvtevpi prrppppqaa nntnsvsnnt niqfpdilsk
askhlpsfqd
181 gsiiapdkis qnlskipplq lieiisnlki lsnqeniqks qlesfldtns ditisvtqal
241 lemgfidysv vtkvlksqvg eapsllssnn tsnsntpvsv irnntplhvp
snevsnnpnn
301 mplnvampmp mstppfiplp lqqqpfgfap pgpfmppaqg psmgqpvlan
qlgqvqqqni
361 sstegpsnan kandsgtinm aklqllpenq qdmikqvltl tpaqiqslps
dqqlmvenfr
421 keyii

YKL018w: GenBank # CAA81853.1
1 mttvsinkpn llkfkhvksf qpqekdcgpv tslnfddngq flltsssndt mqlysatnck
61 fldtiaskky gchsaiftha qneciysstm knfdikylnl etnqylryfs ghgalvndlk
121 mnpvndtfls ssydesvrlw dlkiskpqvi ipslvpncia ydpsglvfal
gnpenfeigl
181 ynlkkiqegp fliikindat fsqwnklefs nngkyllvgs sigkhlifda ftgqqlfeli
241 gtrafpmref ldsgsacftp dgefvlgtdy dgriaiwnhs dsisnkvlrp
qgfipcvshe
301 tcprsiafnp kysmfvtade tvdfyvyde

Glc7 (YER133w): GenBank # AAC03231.1
1 mdsqpvdvdn iidrllevrg skpgqqvdle eneirylcsk arsifikqpi lleleapiki
61 cgdihgqyyd llrlfeyggf ppesnylflg dyvdrgkqsl eticlllayk ikypenffil
121 rgnhecasin riygfydeck rrynikiwkt ftdcfnclpi aaiidekifc mhgglspdln
181 smeqirrvmr ptdipdvgll cdllwsdpdk divgwsendr gvsftfgpdv
vnrflqkqdm
241 elicrahqvv edgyeffskr qlvtlfsapn ycgefdnaga mmsvdesllc
sfqilkpaqk
301 slprqaggrk kk

Ssu72 (YNL222w): GenBank # CAA96125.1
1 mpshrnsnlk fctvcasnnn rsmeshkvlq eagynvssyg tgsavrlpgl
sidkpnvysf
61 gtpyndiynd llsqsadryk sngllqmldr nrrlkkapek wqestkvfdf
vftceercfd
121 avcedlmnrg gklnkivhvi nvdikddden akigskaile ladmlndkie
qcekddipfe
181 dcimdiltew qsshsqlpsl yapsyy

YOR179c: GenBank # CAA99388.1
1 mdlpkdksdr thqrinlnns gtdrtndlyl hivqtfgcie ttatenatkl lmlgdvevei
61 sassvsiewt qksmisqtia dsivimiigl casdknvlse selkernhnv
wkiqelqnlf
121 reqfgdsfsi degigkkenv kngsvtigks katidfstmk lidcnsnplk grvesilsig
181 qklttplc

## Claims

1. Component of the cleavage/polyadenylation machinery of precursor RNA selected from
a) Yeast proteins YGR156w comprising the sequence of Seq ID No. 2, YKL018w comprising the sequence Seq ID No. 3, YOR179c comprising the sequence of Seq ID No. 4 and YKL059c comprising the sequence of Seq ID No. 1.
b) The mammalian homologs of the proteins of a).
c) Derivatives of the above proteins according to a) and b) carrying one or more acid substitutions, deletions and/or additions, and
d) Proteins showing a percent identity of at least 40 percent to any of the proteins of a).

2. Components according to claim 1 **characterized in that** it shows a percent identity of at least 70 percent, more preferably at least 90 percent to any of the proteins of claim 1a).

3. Component according to any of claims 1 or 2, **characterized in that** it is encoded by a nucleic acid sequence which hybridizes to a nucleic acid encoding any of the proteins of claim 1a).

4. Fusion protein comprising at least one of the components of any of claims 1-3.

5. Composition, preferably a protein complex useful for cleaving and/or polyadenylating a nucleic acid, comprising at least one protein selected from the components of claim 1 and Glc7 (YER133w), Ssu72(YNL222w) and Ref2 (YDR195W).

6. Protein complex according to claim 5, **characterized in that** it comprises at least two of the proteins according to claim 5, preferably at least YKL059c, YGR156w, YKL018w, Glc7, Ssu72, YOR179c and Ref2 and the mammalian homologs thereof, respectively.

7. Nucleic acid encoding a component or fusion protein according to any of claims 1 to 4, preferably selected from the nucleic acids encoding a protein selected from YKL059c, YGR156w, YKL018w, Glc7, Ssu72, YOR179c and Ref2 and nucleic acids hybridizing therewith and encoding the mammalian homologs thereof.

8. Construct, preferably a vector construct, comprising a nucleic acid according to claim 7 and at least one further nucleic acid which is normally not associated with the nucleic acid according to claim 7.

9. Host cell containing a nucleic acid of claim 7 and/or a construct of claim 8.

10. A product according to any of claims 1 to 7 as a target for a therapeutic drug.

11. The product according to claim 10 **characterized in that** it is the target for a therapeutic drug selected from an antiviral agent, an anticancer agent, an agent for treating neurological disorders and an agent for treating an autoimmune disease.

12. A process for preparing a component of claim 1, comprising the following steps:
tagging a protein of the mRNA 3' end processing complex (cleavage/polyadenylation machinery) with a sequence that allows affinity purification of the tagged protein,
expressing such protein in a target cell,
isolating the protein complex which is attached to the tagged protein,
disassociating the protein complex and isolating the individual complex members.

13. The process according to claim 12 **characterized in that** the tagged protein comprises two different tags which allow two separate affinity purification steps.

14. The process according to any of claims 12-13, **characterized in that** the two tags are separated by a cleavage site for a protease.

15. Component of the cleavage/polyadenylation machinery of precursors RNA obtainable by a process according to any of claims 11-14.

16. Pharmaceutical composition comprising a product according to any of claims 1 to 8.

17. Kit for processing RNA comprising a product according to any of claims 1 to 8 optionally together with further components such as reagents and working instructions.

18. Kit for the diagnosis of a disease of mammals comprising a product according to any of claims 1 to 8 optionally together with further components such as reagents and working instructions.

19. Antibody binding to a product according to any of claims 1 to 7.

20. A process for the identification and/or preparation of an effector of the cleavage/polyadenylation machinery of precursor RNA comprising the step of bringing into contact a product of any of claims 1 to 7 with a compound, a mixture of compounds or a library of compounds and determining whether the compound or a certain compound of the mixture or library binds to the product and/or effects the product's biological activity and optionally further purifying the compound positively tested as effector.

21. An effector of a component according to claim 1 obtainable by the process according to claim 20.

22. A process for processing RNA comprising the step of bringing into contact a product according to any of claims 1 to 6 with RNA, such that the RNA is processed.

23. A process for modifying the RNA processing activity of a given cell comprising the step of introducing a product of any of claims 1 to 8 and/or an effector of claim 21 and/or an antibody of claim 19 into the cell.

24. Use of a product according to any of claims 1 to 6 for identifying an effector of the cleavage/polyadenylation of precursor RNA.


SEQUENCE LISTING

<110> Cellzome GmbH

<120> Cleavage and polyadenylation complex of precursor mRNA

<130> PTA-1

<140> 01111774.4
<141> 2001-05-15

<160> 7

<170> PatentIn Ver. 2.1

<210> 1
<211> 441
<212> PRT
<213> Saccharomyces cerevisiae

<400> 1
Met Ser Ser Thr Ile Phe Tyr Arg Phe Lys Ser Gln Arg Asn Thr Ser
1               5                   10                  15

Arg Ile Leu Phe Asp Gly Thr Gly Leu Thr Val Phe Asp Leu Lys Arg
            20                  25                  30

Glu Ile Ile Gln Glu Asn Lys Leu Gly Asp Gly Thr Asp Phe Gln Leu
        35                  40                  45

Lys Ile Tyr Asn Pro Asp Thr Glu Glu Glu Tyr Asp Asp Asp Ala Phe
    50                  55                  60

Val Ile Pro Arg Ser Thr Ser Val Ile Val Lys Arg Ser Pro Ala Ile
65                  70                  75                  80

Lys Ser Phe Ser Val His Ser Arg Leu Lys Gly Asn Val Gly Ala Ala
                85                  90                  95

Ala Leu Gly Asn Ala Thr Arg Tyr Val Thr Gly Arg Pro Arg Val Leu
            100                 105                 110

Gln Lys Arg Gln His Thr Ala Thr Thr Thr Ala Asn Val Ser Gly Thr
        115                 120                 125

Thr Glu Glu Glu Arg Ile Ala Ser Met Phe Ala Thr Gln Glu Asn Gln
    130                 135                 140

Trp Glu Gln Thr Gln Glu Glu Met Ser Ala Ala Thr Pro Val Phe Phe
145                 150                 155                 160

Lys Ser Gln Thr Asn Lys Asn Ser Ala Gln Glu Asn Glu Gly Pro Pro
                165                 170                 175

Pro Pro Gly Tyr Met Cys Tyr Arg Cys Gly Gly Arg Asp His Trp Ile
            180                 185                 190

Lys Asn Cys Pro Thr Asn Ser Asp Pro Asn Phe Glu Gly Lys Arg Ile
        195                 200                 205

Arg Arg Thr Thr Gly Ile Pro Lys Lys Phe Leu Lys Ser Ile Glu Ile
    210                 215                 220

Asp Pro Glu Thr Met Thr Pro Glu Glu Met Ala Gln Arg Lys Ile Met
225                 230                 235                 240

Ile Thr Asp Glu Gly Lys Phe Val Val Gln Val Glu Asp Lys Gln Ser
                245                 250                 255

Trp Glu Asp Tyr Gln Arg Lys Arg Glu Asn Arg Gln Ile Asp Gly Asp
            260                 265                 270

Glu Thr Ile Trp Arg Lys Gly His Phe Lys Asp Leu Pro Asp Asp Leu
        275                 280                 285

Lys Cys Pro Leu Thr Gly Gly Leu Leu Arg Gln Pro Val Lys Thr Ser
    290                 295                 300

Lys Cys Cys Asn Ile Asp Phe Ser Lys Glu Ala Leu Glu Asn Ala Leu
305                 310                 315                 320

Val Glu Ser Asp Phe Val Cys Pro Asn Cys Glu Thr Arg Asp Ile Leu
                325                 330                 335

Leu Asp Ser Leu Val Pro Asp Gln Asp Lys Glu Lys Glu Val Glu Thr
            340                 345                 350

Phe Leu Lys Lys Gln Glu Glu Leu His Gly Ser Ser Lys Asp Gly Asn
        355                 360                 365

Gln Pro Glu Thr Lys Lys Met Lys Leu Met Asp Pro Thr Gly Thr Ala
    370                 375                 380

Gly Leu Asn Asn Asn Thr Ser Leu Pro Thr Ser Val Asn Asn Gly Gly
385                 390                 395                 400

Thr Pro Val Pro Pro Val Pro Leu Pro Phe Gly Ile Pro Pro Phe Pro
                405                 410                 415

Met Phe Pro Met Pro Phe Met Pro Pro Thr Ala Thr Ile Thr Asn Pro
            420                 425                 430

His Gln Ala Asp Ala Ser Pro Lys Lys
        435                 440


<210> 2
<211> 425
<212> PRT
<213> Saccharomyces cerevisiae

<400> 2

Met Thr Asp Pro Arg Arg Arg Thr Gly Arg His Phe Leu Thr Pro Glu
1               5                   10                  15

Asn Leu Ser Ser Thr Leu Gln Ile Thr Asn Leu Pro Pro Glu Trp Asn
            20                  25                  30

Gln Asp Ile Ile Thr Ser Val Val Ala Gly Ser Gly Pro Val Ile Asp
        35                  40                  45

Ile Lys Ala Lys Asn Asp Pro Arg Thr Gly Lys Leu Thr Gly Val Leu
    50                  55                  60

Phe Asp Tyr Leu Thr Ser Lys Asp Cys Lys Arg Ala Trp Glu Ile Leu
65                  70                  75                  80

Asn Arg Ile Glu Asn Phe Pro Val Lys Ile Glu Gln Ile Ile Pro Pro
                85                  90                  95

Asn Tyr Lys Asp His Leu Arg Glu Thr Ala Asn Lys Asn Ser Gln Lys
            100                 105                 110

Gln Val Leu Gln Leu Asn Arg Asp Ser Tyr Pro Phe Glu Ala Gly Leu
        115                 120                 125

Glu Leu Pro Phe Glu Met Val Thr Glu Val Pro Ile Pro Arg Arg Pro
    130                 135                 140

Pro Pro Pro Gln Ala Ala Asn Asn Thr Asn Ser Val Ser Asn Asn Thr
145                 150                 155                 160

Asn Ile Gln Phe Pro Asp Ile Leu Ser Lys Ala Ser Lys His Leu Pro
                165                 170                 175

Ser Phe Gln Asp Gly Ser Ile Ile Ala Pro Asp Lys Ile Ser Gln Asn
            180                 185                 190

Leu Ser Lys Ile Pro Pro Leu Gln Leu Ile Glu Ile Ile Ser Asn Leu
        195                 200                 205

Lys Ile Leu Ser Asn Gln Glu Asn Ile Gln Lys Ser Gln Leu Glu Ser
    210                 215                 220

Phe Leu Asp Thr Asn Ser Asp Ile Thr Ile Ser Val Thr Gln Ala Leu
225                 230                 235                 240

Leu Glu Met Gly Phe Ile Asp Tyr Ser Val Val Thr Lys Val Leu Lys
                245                 250                 255

Ser Gln Val Gly Glu Ala Pro Ser Leu Leu Ser Ser Asn Asn Thr Ser
            260                 265                 270

Asn Ser Asn Thr Pro Val Ser Val Ile Arg Asn Asn Thr Pro Leu His
        275                 280                 285

Val Pro Ser Asn Glu Val Ser Asn Asn Pro Asn Asn Met Pro Leu Asn
    290                 295                 300

Val Ala Met Pro Met Pro Met Ser Thr Pro Pro Phe Ile Pro Leu Pro
305                 310                 315                 320

Leu Gln Gln Gln Pro Phe Gly Phe Ala Pro Pro Gly Pro Phe Met Pro
                325                 330                 335

Pro Ala Gln Gly Pro Ser Met Gly Gln Pro Val Leu Ala Asn Gln Leu
            340                 345                 350

Gly Gln Val Gln Gln Gln Asn Ile Ser Ser Thr Glu Gly Pro Ser Asn
        355                 360                 365

Ala Asn Lys Ala Asn Asp Ser Gly Thr Ile Asn Met Ala Lys Leu Gln
    370                 375                 380

Leu Leu Pro Glu Asn Gln Gln Asp Met Ile Lys Gln Val Leu Thr Leu
385                 390                 395                 400

Thr Pro Ala Gln Ile Gln Ser Leu Pro Ser Asp Gln Gln Leu Met Val
                405                 410                 415

Glu Asn Phe Arg Lys Glu Tyr Ile Ile
            420                 425


<210> 3
<211> 329
<212> PRT
<213> Saccharomyces cerevisiae

<400> 3
Met Thr Thr Val Ser Ile Asn Lys Pro Asn Leu Leu Lys Phe Lys His
1               5                   10                  15

Val Lys Ser Phe Gln Pro Gln Glu Lys Asp Cys Gly Pro Val Thr Ser
            20                  25                  30

Leu Asn Phe Asp Asp Asn Gly Gln Phe Leu Leu Thr Ser Ser Ser Asn
        35                  40                  45

Asp Thr Met Gln Leu Tyr Ser Ala Thr Asn Cys Lys Phe Leu Asp Thr
    50                  55                  60

Ile Ala Ser Lys Lys Tyr Gly Cys His Ser Ala Ile Phe Thr His Ala
65                  70                  75                  80

Gln Asn Glu Cys Ile Tyr Ser Ser Thr Met Lys Asn Phe Asp Ile Lys
                85                  90                  95

Tyr Leu Asn Leu Glu Thr Asn Gln Tyr Leu Arg Tyr Phe Ser Gly His
            100                 105                 110

Gly Ala Leu Val Asn Asp Leu Lys Met Asn Pro Val Asn Asp Thr Phe
        115                 120                 125

Leu Ser Ser Ser Tyr Asp Glu Ser Val Arg Leu Trp Asp Leu Lys Ile
    130                 135                 140

Ser Lys Pro Gln Val Ile Ile Pro Ser Leu Val Pro Asn Cys Ile Ala
145                 150                 155                 160

Tyr Asp Pro Ser Gly Leu Val Phe Ala Leu Gly Asn Pro Glu Asn Phe
                165                 170                 175

Glu Ile Gly Leu Tyr Asn Leu Lys Lys Ile Gln Glu Gly Pro Phe Leu
            180                 185                 190

Ile Ile Lys Ile Asn Asp Ala Thr Phe Ser Gln Trp Asn Lys Leu Glu
        195                 200                 205

Phe Ser Asn Asn Gly Lys Tyr Leu Leu Val Gly Ser Ser Ile Gly Lys
    210                 215                 220

His Leu Ile Phe Asp Ala Phe Thr Gly Gln Gln Leu Phe Glu Leu Ile
225                 230                 235                 240

Gly Thr Arg Ala Phe Pro Met Arg Glu Phe Leu Asp Ser Gly Ser Ala
                245                 250                 255

Cys Phe Thr Pro Asp Gly Glu Phe Val Leu Gly Thr Asp Tyr Asp Gly
            260                 265                 270

Arg Ile Ala Ile Trp Asn His Ser Asp Ser Ile Ser Asn Lys Val Leu
        275                 280                 285

Arg Pro Gln Gly Phe Ile Pro Cys Val Ser His Glu Thr Cys Pro Arg
    290                 295                 300

Ser Ile Ala Phe Asn Pro Lys Tyr Ser Met Phe Val Thr Ala Asp Glu
305                 310                 315                 320

Thr Val Asp Phe Tyr Val Tyr Asp Glu
                325


<210> 4
<211> 188
<212> PRT
<213> Saccharomyces cerevisiae

<400> 4
Met Asp Leu Pro Lys Asp Lys Ser Asp Arg Thr His Gln Arg Ile Asn
  1               5                  10                  15

Leu Asn Asn Ser Gly Thr Asp Arg Thr Asn Asp Leu Tyr Leu His Ile
             20                  25                  30

Val Gln Thr Phe Gly Cys Ile Glu Thr Thr Ala Thr Glu Asn Ala Thr
         35                  40                  45

Lys Leu Leu Met Leu Gly Asp Val Glu Val Glu Ile Ser Ala Ser Ser
    50                  55                  60

Val Ser Ile Glu Trp Thr Gln Lys Ser Met Ile Ser Gln Thr Ile Ala
65                  70                  75                  80

Asp Ser Ile Val Ile Met Ile Ile Gly Leu Cys Ala Ser Asp Lys Asn
                85                  90                  95

Val Leu Ser Glu Ser Glu Leu Lys Glu Arg Asn His Asn Val Trp Lys
            100                 105                 110

Ile Gln Glu Leu Gln Asn Leu Phe Arg Glu Gln Phe Gly Asp Ser Phe
        115                 120                 125

Ser Ile Asp Glu Gly Ile Gly Lys Lys Glu Asn Val Lys Asn Gly Ser
    130                 135                 140

Val Thr Ile Gly Lys Ser Lys Ala Thr Ile Asp Phe Ser Thr Met Lys
145                 150                 155                 160

Leu Ile Asp Cys Asn Ser Asn Pro Leu Lys Gly Arg Val Glu Ser Ile
                165                 170                 175

Leu Ser Ile Gly Gln Lys Leu Thr Thr Pro Leu Cys
            180                 185

<210> 5
<211> 533
<212> PRT
<213> Saccharomyces cerevisiae

<400> 5
Met Ser Ala Pro Val Pro Gln Leu Val Asn Ile Ser His Ala Leu Gln
1               5                   10                  15

Ala Ser Thr Ile Gln Gln Ile Arg Leu Asp Met Val Asp Phe Asn Lys
            20                  25                  30

Asp Cys Lys Leu Ser Ser Ile Gln Leu Ala Arg Ile Asp Lys Tyr Ile
        35                  40                  45

Asp Ser Leu Gln Ala Ala Leu Asn Gln Phe Thr Lys Asp Asn Leu His
    50                  55                  60

Ile Glu Arg Lys Glu Lys Asn Val Thr Glu Ala Asp Ile Gln Leu Tyr
65                  70                  75                  80

Ser Gly Leu Lys Ser Met Tyr Leu Asp Tyr Leu Asn Gln Leu Ile Lys
                85                  90                  95

Leu Lys His Glu Lys Gln His His Ser Thr Pro Pro Ile Ala Asn Asp
            100                 105                 110

Val Ser Leu Asp Phe Phe Val Asn Gln Leu Pro Lys Phe Ser Pro Glu
        115                 120                 125

Glu Arg Lys Asn Tyr Ile Asp Asn Leu Ile Leu Asn Lys Asn Ser His
    130                 135                 140

Asn Arg Leu Ser Lys Met Asp Gly Leu Val Asp Ala Val Ile Asn Leu
145                 150                 155                 160

Cys Val Leu Asp Thr Ser Val Ala Glu Asn Val Arg Ser Tyr Met Lys
                165                 170                 175

Leu Leu Asp Thr Leu Gly Phe Gln Lys Gly Ser Asn Ser Thr Gly Thr
            180                 185                 190

Lys Ala Asn Leu Lys Lys Lys Leu Ala Ser Ser Lys Ala Lys Ile Lys
        195                 200                 205

Asp Ser Glu Lys Glu Lys Glu Lys Glu Lys Asp Lys Ser Lys Val Lys
    210                 215                 220

Met Lys Thr Lys Leu Lys Pro Ser Pro Leu Leu Asn Asn Asp Asp Lys
225                 230                 235                 240

Asn Ser Ser Pro Ser Pro Thr Ala Ser Thr Ser Ser Met Lys Lys Leu
                245                 250                 255

Lys Ser Gly Leu Phe Asn Lys Asn Glu Ala Lys Ser Thr Glu Ser Leu
            260                 265                 270

Pro Thr Ser Ser Lys Lys Lys Leu Ser Phe Ser Lys Tyr Leu Asn Lys
        275                 280                 285

Asp Asp Ala Asp Met Thr Lys Leu Gly Thr Lys Arg Ser Ile Asp Val
    290                 295                 300

Asp Phe Lys Val Asn Pro Glu Ala Ser Thr Val Ala Ser Asn Ile Ile
305                 310                 315                 320

Ser Ser Ser Thr Ser Gly Ser Ser Thr Thr Thr Val Ala Thr Pro Ala
                325                 330                 335

Ser Ser Glu Glu Pro Leu Lys Lys Lys Thr Lys Ile Ser Val Gln Asp
            340                 345                 350

Ser Asn Val Gln Ser Ile Leu Arg Asn Gly Lys Pro Lys Lys Ala Arg
        355                 360                 365

Ile Ser Ser Ile Lys Phe Leu Asp Asp Ser Gln Leu Ile Lys Val Tyr
    370                 375                 380

Gly Asp Asp Leu Pro Asn Gln Gly Leu Gln Val Ser Pro Thr Gln Leu
385                 390                 395                 400

Lys Lys Ile Leu Lys Pro Phe Lys Glu Gly Glu Pro Lys Glu Ile Ile
                405                 410                 415

Leu Phe Glu Asp Met Ser Ile Lys Leu Lys Pro Leu Asp Leu Met Phe
            420                 425                 430

Leu Lys Asn Thr Asn Ser Asp Asp Tyr Met Asp Ile Ser Glu Thr Lys
        435                 440                 445

Gly Gly Pro Ile His Cys Glu Thr Arg Thr Pro Leu Ile Tyr Arg Lys
    450                 455                 460

Asn Phe Asn His Phe Asn Pro Asp Leu Asn Lys Arg Pro Pro Arg Glu
465                 470                 475                 480

Pro Ile Glu Phe Asp Leu Asn Gly Asn Thr Asn Ser Thr Pro Thr Ile
                485                 490                 495

Ala Lys Ala Phe Gly Lys Asn Ser Leu Leu Leu Arg Lys Asp Arg Gly
            500                 505                 510

Gly Leu Pro Tyr Lys His Val Pro Ile Val Lys Arg Asn Lys Tyr Pro
        515                 520                 525

Pro Arg Pro Val His
    530


<210> 6
<211> 312
<212> PRT
<213> Saccharomyces cerevisiae

<400> 6
Met Asp Ser Gln Pro Val Asp Val Asp Asn Ile Ile Asp Arg Leu Leu
  1               5                  10                  15

Glu Val Arg Gly Ser Lys Pro Gly Gln Gln Val Asp Leu Glu Glu Asn
             20                  25                  30

Glu Ile Arg Tyr Leu Cys Ser Lys Ala Arg Ser Ile Phe Ile Lys Gln
         35                  40                  45

Pro Ile Leu Leu Glu Leu Glu Ala Pro Ile Lys Ile Cys Gly Asp Ile
     50                  55                  60

His Gly Gln Tyr Tyr Asp Leu Leu Arg Leu Phe Glu Tyr Gly Gly Phe
 65                  70                  75                  80

Pro Pro Glu Ser Asn Tyr Leu Phe Leu Gly Asp Tyr Val Asp Arg Gly
                 85                  90                  95

Lys Gln Ser Leu Glu Thr Ile Cys Leu Leu Leu Ala Tyr Lys Ile Lys
            100                 105                 110

Tyr Pro Glu Asn Phe Phe Ile Leu Arg Gly Asn His Glu Cys Ala Ser
        115                 120                 125

Ile Asn Arg Ile Tyr Gly Phe Tyr Asp Glu Cys Lys Arg Arg Tyr Asn
130                 135                 140

Ile Lys Leu Trp Lys Thr Phe Thr Asp Cys Phe Asn Cys Leu Pro Ile
145                 150                 155                 160

Ala Ala Ile Ile Asp Glu Lys Ile Phe Cys Met His Gly Gly Leu Ser
                165                 170                 175

Pro Asp Leu Asn Ser Met Glu Gln Ile Arg Arg Val Met Arg Pro Thr
            180                 185                 190

Asp Ile Pro Asp Val Gly Leu Leu Cys Asp Leu Leu Trp Ser Asp Pro
        195                 200                 205

Asp Lys Asp Ile Val Gly Trp Ser Glu Asn Asp Arg Gly Val Ser Phe
    210                 215                 220

Thr Phe Gly Pro Asp Val Val Asn Arg Phe Leu Gln Lys Gln Asp Met
225                 230                 235                 240

Glu Leu Ile Cys Arg Ala His Gln Val Val Glu Asp Gly Tyr Glu Phe
                245                 250                 255

Phe Ser Lys Arg Gln Leu Val Thr Leu Phe Ser Ala Pro Asn Tyr Cys
            260                 265                 270

Gly Glu Phe Asp Asn Ala Gly Ala Met Met Ser Val Asp Glu Ser Leu
        275                 280                 285

Leu Cys Ser Phe Gln Ile Leu Lys Pro Ala Gln Lys Ser Leu Pro Arg
    290                 295                 300

Gln Ala Gly Gly Arg Lys Lys Lys
305                 310


<210> 7
<211> 206
<212> PRT
<213> Saccharomyces cerevisiae

<400> 7
Met Pro Ser His Arg Asn Ser Asn Leu Lys Phe Cys Thr Val Cys Ala
1               5                   10                  15

Ser Asn Asn Asn Arg Ser Met Glu Ser His Lys Val Leu Gln Glu Ala
            20                  25                  30

Gly Tyr Asn Val Ser Ser Tyr Gly Thr Gly Ser Ala Val Arg Leu Pro
        35                  40                  45

Gly Leu Ser Ile Asp Lys Pro Asn Val Tyr Ser Phe Gly Thr Pro Tyr
    50                  55                  60

Asn Asp Ile Tyr Asn Asp Leu Leu Ser Gln Ser Ala Asp Arg Tyr Lys
65                  70                  75                  80

Ser Asn Gly Leu Leu Gln Met Leu Asp Arg Asn Arg Arg Leu Lys Lys
                85                  90                  95

Ala Pro Glu Lys Trp Gln Glu Ser Thr Lys Val Phe Asp Phe Val Phe
            100                 105                 110

Thr Cys Glu Glu Arg Cys Phe Asp Ala Val Cys Glu Asp Leu Met Asn
        115                 120                 125

Arg Gly Gly Lys Leu Asn Lys Ile Val His Val Ile Asn Val Asp Ile
    130                 135                 140

Lys Asp Asp Asp Glu Asn Ala Lys Ile Gly Ser Lys Ala Ile Leu Glu
145                 150                 155                 160

Leu Ala Asp Met Leu Asn Asp Lys Ile Glu Gln Cys Glu Lys Asp Asp
                165                 170                 175

Ile Pro Phe Glu Asp Cys Ile Met Asp Ile Leu Thr Glu Trp Gln Ser
            180                 185                 190

Ser His Ser Gln Leu Pro Ser Leu Tyr Ala Pro Ser Tyr Tyr
        195                 200                 205